# EUROPÄISCHE PATENTSCHRIFT

(11) **EP 0 850 905 B1**
(45) Veröffentlichungstag und Bekanntmachung des Hinweises auf die Patenterteilung: **18.07.2001**
(21) Anmeldenummer: 97118801.6
(22) Anmeldetag: 29.10.1997
(51) Int. Cl.: C07C 29/16, C07C 45/50, C07C 29/141, C07C 31/125, C07C 47/02

(54) **Verfahren zur Herstellung von höheren Oxo-Alkoholen**
Process for the preparation of higher oxo-alcohols
Procédé pour la préparation des oxo-alcools supérieurs

(30) Priorität: 24.12.1996 DE 19654340
(43) Veröffentlichungstag der Anmeldung: 01.07.1998
(73) Patentinhaber: Degussa AG, 40474 Düsseldorf (DE)
(72) Erfinder: Gubisch, Dietmar, Dr., 45772 Marl (DE); Armbrust, Klaus, 48249 Dülmen (DE); Kaizik, Alfred, Dr., 45772 Marl (DE); Scholz, Bernhard, Dr., 45768 Marl (DE); Nehring, Rudolf, Dr., 45772 Marl (DE)

(56) Entgegenhaltungen:
- DE-A- 2 139 630
- DE-A- 2 206 252
- DE-A- 2 244 373

## Beschreibung

Die vorliegende Erfindung betrifft ein Verfahren zur Herstellung von Alkoholen mit 7 bis 18 C-Atomen durch Hydroformylierung der entsprechenden Olefine mit Synthesegas in Gegenwart einer einen Kobaltkatalysator enthaltenden organischen Phase bei Temperaturen von 50 bis 220 °C und Drücken von 100 bis 400 bar und anschließende Hydrierung der so erhaltenen Aldehyde, wobei der Kobaltkatalysator durch Umsetzen einer wäßrigen Kobaltsalzlösung in Anwesenheit eines nicht oder nur wenig mit Wasser mischbaren organischen Lösungsmittels mit Synthesegas gebildet und die den Kobaltkatalysator enthaltende organische Phase durch Extraktion des gebildeten Kobaltkatalysators aus der wäßrigen Phase mit einem nicht oder nur wenig mit Wasser mischbaren organischen Extraktionsmittel erhalten wird.

Die Hydroformylierung von Olefinen mit Kohlenmonoxid und Wasserstoff zu um ein C-Atom reicheren Aldehyden in Gegenwart von Übergangsmetall-Katalysatoren, wie beispielsweise Kobalt- und Rhodiumverbindungen, ist als Oxosynthese bekannt. Im allgemeinen wird bei der Hydroformylierung von Olefinen zu Aldehyden ein hoher Anteil an geradkettigen Aldehyden - den Zwischenprodukten bei der Herstellung der wirtschaftlich bedeutenden Weichmacheralkohole für Kunststoffe und Waschmittelalkohole - angestrebt.

Während lineare und endständige Olefine (sog. α-Olefine) mit phosphinmodifizierten Rhodium- bzw. Kobalt-Katalysatoren sehr gut hydroformylierbar sind (J. Falbe, Ed: "New Synhesis With Carbon Monoxide", Springer -Verlag, Berlin 1980, S. 55 ff.), werden für wenig reaktive, für innenständige sowie für innenständige und verzweigte Olefine vorzugsweise unmodifizierte Kobalt- und Rhodium-Katalysatoren eingesetzt.

In Gegenwart von modifizierten Katalysatoren werden innenständige und verzweigte Olefine sehr langsam bzw. nur zum Teil hydroformyliert. Dies schließt eine mögliche Verwendung von modifizierten Katalysatoren für eine wirtschaftliche Hydroformylierung von innenständigen und verzweigten Olefinen aus.

Die Hydroformylierung von polymeren und isomeren Olefingemischen, die endständige und innenständige sowie innenständige und verzweigte Olefine enthalten, wird vorteilhaft mit unmodifzierten Kobalt-Katalysatoren durchgeführt. Im Vergleich zu Rhodium-Katalysatoren werden mit Kobalt-Katalysatoren ausgehend von einem gleichen Ausgangsolefin höhere Ausbeuten an den besonders begehrten geradkettigen Aldehyden erhalten.

Beispiele für typische polymere und isomere Olefingemische, die vorzugsweise durch kobaltkatalysierte Hydroformylierung zu entsprechenden Oxo-Aldehyden umgesetzt werden, sind die Dimere, Trimere und Tetramere des Propens, der n-Butene (1- und 2-Buten) und des iso-Butens.

Nach den bekannten Verfahren wird die kobaltkatalysierte Hydroformylierung als mehrstufiger Prozeß durchgeführt, der folgende vier Verfahrensstufen enthält: die Herstellung des Katalysators (Vorcarbonylierung), die Katalysatorextraktion, die Olefin-Hydroformylierung und die Entfernung des Katalysators aus dem Reaktionsprodukt (Entkobaltung).
Seit der Entwicklung der Oxosynthese wurden die einzelnen Verfahrensstufen der kobaltkatalysierten Hydroformylierung fortwährend verbessert und modifizert.

In der ersten Verfahrensstufe, der Vorcarbonylierung, wird ausgehend von einer wäßrigen Kobaltsalzlösung durch Umsetzung mit Kohlenmonoxid und Wasserstoff der für die Hydroformylierung benötigte Katalysatorkomplex (HCo(CO)₄) hergestellt. Nach DE-OS 21 39 630 wird die Vorcarbonylierung vorzugsweise bei Temperaturen von 100 bis 160 °C und unter Synthesegas-Drücken von 200 bis 300 bar in Gegenwart von Aktivkohle, Zeolithen oder basischen Ionenaustauschern, die mit Kobaltcarbonylen beladen sind, durchgeführt.

Die DE-OS 22 44 373 beschreibt ein verbessertes, kontinuierliches Carbonylierungsverfahren, bei dem eine deutliche Verkürzung der Reaktionszeit dadurch erreicht wird, indem die Edukte Synthesegas und wäßrige Kobaltsalzlösung im Gleichstrom in Gegenwart von mit Wasser schwer oder nicht mischbaren sauerstoffhaltigen organischen Lösungsmitteln durch eine Zone, in der eine turbulente Strömung aufrechterhalten wird, geleitet werden. Als vorteilhafte Ausführung wird die Verwendung eines Druckturbulenzrohres zur Aufrechterhaltung der turbulenten Strömung und der Zusatz von Alkoholen oder Aldehyden mit 4 bis 10 Kohlenstoffatomen als organisches Lösungsmittel aufgeführt.

In der zweiten Verfahrensstufe, der Katalysatorextraktion, wird der in der ersten Verfahrensstufe hergestellte Kobaltkatalysator aus der wäßrigen Phase mit einer organischen Phase, vorzugsweise mit dem zu hydroformylierenden Olefin, extrahiert. Gemäß DE-OS 21 06 252 ist es zweckmäßig, neben dem Olefin die Reaktions- und Nebenprodukte der Hydroformylierung, sofern sie wasserunlöslich und unter den gewählten Reaktionsbedingungen flüssig sind, zur Katalysatorextraktion einzusetzen. Die Katalysatorextraktion wird vorzugsweise im Gegenstrom bei Temperaturen von 20 bis 100 °C und unter Synthesegas-Drücken von 100 bis 400 bar durchgeführt. Nach der Phasentrennung wird die mit dem Kobaltkatalysator beladene organische Phase der dritten Verfahrensstufe, der Hydroformylierung zugeführt.

Aus DE-OS 21 39 630 ist bekannt, daß in der dritten Verfahrensstufe, der Hydroformylierung, mit dem Kobaltkatalysator beladene Olefine in einem Hochdruckreaktor mit Synthesegas bei Temperaturen zwischen 70 und 170 °C und Drücken von 100 bis 400 bar zu den entsprechenden Aldehyden hydroformyliert werden können. Ein Teil des gebildeten Aldehydes kann unter den Hydroformylierungsbedingungen, insbesondere bei hohen Temperaturen, zum Alkohol hydriert werden.
Der Reaktoraustrag, der neben den Wertprodukten Aldehyd und Alkohol zusätzlich Nebenprodukte, nicht hydroformyliertes Restolefin und Kobalt-Katalysator enthält, wird bis auf 1 bis 15 bar entspannt und anschließend der Katalysatoraufarbeitungsstufe zugeführt.

In der vierten Verfahrenstufe, der Entkobaltung, wird die organische Phase des Reaktoraustrages von den Kobaltcarbonylkomplexen in Gegenwart von komplexfreiem Prozeßwasser durch Behandlung mit Sauerstoff oder Luft befreit. Gemäß WO 93/24438 wird die Entkobaltung bei Temperaturen von 60 bis 100 °C und Drücken von 1 bis 20 bar durchgeführt. Dabei wird der Kobaltkatalysator oxidativ zerstört und die resultierenden Kobaltsalze in die wäßrige: Phase zurückextrahiert. Die resultierende wäßrige Kobaltsalzlösung aus der Entkobaltung wird in die erste Verfahrensstufe, die Vorcarbonylierung, zurückgeführt.

Eine weitere Ausführungsform ist in WO 93/24437 und EP-OS 0 183 546 beschrieben. Hier wird vor der oxidativen Zerstörung des Kobaltkatalysators eine Gaswäsche mit Synthesegas oder Stickstoff durchgeführt.

Die Reaktionsprodukte in der zurückbleibenden organischen Phase werden nach Abtrennen der Gasphase in weiteren Aufarbeitungsstufen, wie Hydrierung und Destillation, in die entsprechenden Alkohole überführt.

Die bekannten, mehrstufigen Herstellungsverfahren von Oxo-Aldehyden in Gegenwart von Kobalt-Katalysatoren weisen eine Reihe von technischen Nachteilen auf. So sind für die Herstellung des für die Hydroformylierung benötigten Kobaltkatalysators zwei technisch aufwendige Verfahrensstufen - Vorcarbonylierung und Katalysatorextraktion - erforderlich. Bedingt durch die in den beiden Verfahrensstufen ablaufenden Stoffübergangsvorgänge, Gas/Flüssigkeit-Stoffübergang bei der Vorcarbonylierung und Flüssigkeit/Flüssigkeit-Stoffübergang bei der Katalysatorextraktion, sind zwei voneinander getrennte druckfeste Apparate, wie z. B. Rührkessel oder Füllkörperkolonnen, erforderlich. Die eigentliche Hydroformylierung findet anschließend wiederum in einem separaten Druckreaktor statt. Das Entfernen des Kobaltkatalysators ist an einen weiteren Anlagenteil gebunden.

Die bekannten mehrstufigen Hydroformylierungsverfahren setzen demzufolge neben einem hohen verfahrenstechnischen Aufwand ein sehr hohes Investment voraus.

Der vorliegenden Erfindung liegt daher die Aufgabe zugrunde, ein neues Hydroformylierungsverfahren von Olefinen mit anschließender Hydrierung der erhaltenen Aldehyde zu entwickeln, das verfahrenstechnisch einfacher durchzuführen und wirtschaftlicher ist.

Es wurde nun überraschenderweise gefunden, daß die Bildung des Kobaltkatalysators, die Extraktion des gebildeten Kobaltkatalysators in die organische Phase und die Hydroformylierung der entsprechenden Olefine in einem 1-Stufen-Prozeß durchgeführt werden können.

Es werden also die ersten drei Prozeßschritte der herkömmlichen Verfahren, die Vorcarbonylierung, die Katalysatorextraktion und die Hydroformylierung in einem 1-Stufen-Prozeß, vorzugsweise in einem Reaktor, durchgeführt.
Die aufwendige apparative Trennung der Verfahrensstufen entfällt somit. Dies ist von besonderem wirtschaftlichen Interesse, da die Reduzierung der Prozeßstufen eine erhebliche Reduktion der Investitionskosten bedeutet. Im erfindungsgemäßen Verfahren laufen Vorcarbonylierung, Extraktion und Hydroformylierung nebeneinander und zum Teil in situ ab. Weiterhin wird der erfindungsgemäße 1-Stufen-Prozeß bevorzugt kontinuierlich durchgeführt, wobei die nach der Entkobaltung anfallende Kobaltsalzlösung vorzugsweise im Kreis gefahren wird.

Gegenstand der vorliegenden Erfindung ist daher ein Verfahren zur Herstellung von Alkoholen mit 7 bis 18 C-Atomen durch Hydroformylierung der entsprechenden Olefine mit Synthesegas in Gegenwart einer einen Kobaltkatalysator enthaltenden organischen Phase bei Temperaturen von 50 bis 220° C und Drücken von 100 bis 400 bar und anschließende Hydrierung der so erhaltenen Aldehyde, wobei der Kobaltkatalysator durch Umsetzen einer wäßrigen Kobaltsalzlösung in Anwesenheit eines nicht oder nur wenig mit Wasser mischbaren organischen Lösungsmittels mit Synthesegas gebildet und die den Kobaltkatalysator enthaltende organische Phase durch Extraktion des gebildeten Kobaltkatalysators aus der wäßrigen Phase mit einem nicht oder nur wenig mit Wasser mischbaren organischen Extraktionsmittel erhalten wird, das dadurch gekennzeichnet ist, daß die Bildung des Kobaltkatalysators, die Extraktion des gebildeten Kobaltkatalysators in die organische Phase und die Hydroformylierung der entsprechenden Olefine in einem 1-Stufen-Prozeß durchgeführt werden.

Vorzugsweise werden die Bildung des Kobaltkatalysators, die Extraktion des gebildeten Kobaltkatalysators in die organische Phase und die Hydroformylierung der entsprechenden Olefine in einem einzigen Reaktor durchgeführt.

Außerdem ist Gegenstand der vorliegenden Erfindung die Verwendung der erfindungsgemäß hergestellten Alkohole zur Herstellung von Carbonsäureestern als Weichmacher für Kunststoffe.

Das erfindungsgemäße Verfahren wird bevorzugt kontinuierlich durchgeführt

Als Kobaltsalze werden bevorzugt wasserlösliche Kobaltsalze, wie beispielsweise Formiate und Acetate, verwendet. Besonderes bewährt hat sich Kobaltacetat, das vorzugsweise als wäßrige Lösung mit einem Kobalt-Gehalt von 0.2 bis 2 Gew.-%, besonders vorzugsweise von 0.5 bis 1.5 Gew.-%, gerechnet als Metall, eingesetzt wird.

Das organische Lösungsmittel kann das zu hydroformylierende Olefin und/oder ein Aldehyd und/oder ein Alkohol sein, wobei es sich bei dem Aldehyd und dem Alkohol vorzugsweise um die während der Hydroformylierung gebildeten Reaktionsprodukte handelt.

Also ist das nicht oder nur wenig mit Wasser mischbare organische Lösungsmittel vorzugsweise ein Olefin und/oder ein Aldehyd und/oder ein Alkohol, besonders bevorzugt das Reaktionsprodukt aus dem 1-Stufen-Prozeß.

Das zur Extraktion des Kobaltkatalysators aus der wäßrigen Phase benötigte Extraktionsmittel kann jedes nicht oder nur schwer mit Wasser mischbare organische Lösungsmittel sein, sofern es eine ausreichende Löslichkeit für den Kobaltkatalysator aufweist. Bevorzugt wird jedoch ein Gemisch aus dem zu hydroformylierenden Olefin und den während der Hydroformylierung gebildeten Aldehyden und/oder Alkoholen eingesetzt.

Also wird als nicht oder nur wenig mit Wasser mischbares organisches Extraktionsmittel vorzugsweise ein Olefin und/oder ein Aldehyd und/oder ein Alkohol, besonders vorzugsweise das Reaktionsprodukt aus dem 1-Stufen-Prozeß, eingesetzt.

Geeigneterweise sind das nicht oder nur wenig mit Wasser mischbare organische Lösungsmittel und das nicht oder nur wenig mit Wasser mischbare organische Extraktionsmittel identisch.

Eine besondere Bedeutung wird bei dem erfindungsgemäßen Verfahren der Dosierung der Ausgangsstoffe in den Reaktor des 1-Stufen-Prozesses beigemessen. Die Dosiervorrichtung soll eine gute Phasenvermischung und die Erzeugung einer möglichst hohen Phasenaustauschfläche gewährleisten.
Für die Dosierung der Ausgangsstoffe können die in der Technik bekannten Dosiervorrichtungen, wie z. B. mit Füllkörpern befüllte Turbulenzröhren oder Mischdüsen für Mehrphasensysteme, eingesetzt werden. Die Dosierung wird vorzugsweise mit einer Mischdüse unter Aufrechterhaltung einer turbulenten Strömung durchgeführt.

Die wässrige Kobaltsalzlösung, Olefin, Synthesegas, ein nicht oder nur wenig mit Wasser mischbares organisches Lösungsmittel und ein nicht oder nur wenig mit Wasser mischbares organisches Extraktionsmittel können gleichzeitig, insbesondere mittels einer Mischdüse, in den Reaktor des 1-Stufen-Prozesses eingebracht werden.

In einer anderen Variante des erfindungsgemäßen Verfahrens können ein nicht oder nur wenig mit Wasser mischbares organisches Lösungsmittel und ein nicht oder nur wenig mit Wasser mischbares organisches Extraktionsmittel im Reaktor des 1-Stufen-Prozesses vorgelegt und die wässrige Kobaltsalzlösung, Olefin und Synthesegas gleichzeitig, insbesondere mittels einer Mischdüse, in den Reaktor des 1-Stufen-Prozesses eingebracht werden.

In einer weiteren Ausführungsform der vorliegenden Erfindung kann ein nicht oder nur wenig mit Wasser mischbares organisches Extraktionsmittel im Reaktor des 1-Stufen-Prozesses vorgelegt und die wässrige Kobaltsalzlösung, Olefin, Synthesegas und ein nicht oder nur wenig mit Wasser mischbares organisches Lösungsmittel können gleichzeitig, insbesondere mittels einer Mischdüse, in den Reaktor des 1-Stufen-Prozesses eingebracht werden.

Vorzugsweise werden die wässrige Kobaltsalzlösung, Olefin und Synthesegas gleichzeitig, besonders vorzugsweise mittels einer Mischdüse, in den Reaktor des 1-Stufen-Prozesses eingebracht.

Der 1-Stufen-Prozeß, der die Vorcarbonylierung, die Katalysatorextraktion und die Hydroformylierung umfaßt, kann in einem Reaktor bei Temperaturen von 100 bis 250 °C und unter Drücken von 100 bis 400 bar durchgeführt werden. Besonderes bewährt haben sich Temperaturen von 160 bis 220 °C und Synthesegas-Drücke von 200 bis 300 bar. Das Volumenverhältnis von Kohlenmonoxid zu Wasserstoff im Synthesegas liegt im allgemeinen zwischen 2 : 1 und 1 : 2, insbesondere bei 1 : 1. Das Synthesegas wird vorteilhaft im geringen Überschuß zu der stöchiometrisch benötigten Menge verwendet.

Der 1-Stufen-Prozeß kann beispielsweise in den allgemein bekannten zylindrischen, senkrecht stehenden Hochdruck-Blasensäulenreaktoren, ohne oder mit eingebautem koaxialen Einsteckrohr, durchgeführt werden.

In einer bevorzugten Ausführungsform des erfindungsgemäßen Verfahrens wird der Reaktorraum des 1-Stufen-Prozesses durch mindestens eine Trennvorrichtung unterteilt.

Diese Trennvorrichtung kann beispielsweise eine Lochplatte oder ein Siebboden sein und wird geeigneterweise senkrecht zur Fließrichtung des Reaktanden- und Produktenstromes angeordnet.

Durch die Reaktorkaskadierung wird die Rückvermischung gegenüber der einfachen Blasensäule stark vermindert und das Strömungsverhalten dem eines Rohrreaktors angenähert. Diese verfahrenstechnische Maßnahme hat zur Folge, daß sowohl die Raum-Zeit-Ausbeute als auch die Selektivität der Hydroformylierung verbessert werden.

In einer geeigneten Ausführungsform des erfindungsgemäßen Verfahrens kann der Reaktorausaustrag (organische und wäßrige Phase), wobei die wäßrige Phase ganz oder teilweise am Boden des Reaktors abgezogen werden kann, nach Verlassen des Reaktors auf 10 bis 15 bar entspannt und in die zur Entfernung des Kobaltkatalysators nötige Entkobaltung geleitet werden. In der Entkobaltungsstufe kann der Produktaustrag in Gegenwart von wäßriger, saurer Kobaltsalzlösung (Prozeßwasser) mit Luft oder Sauerstoff bei Temperaturen von 50 bis 180 °C von Kobaltcarbonylkomplexen befreit werden. Die wäßrige, saure Kobaltsalzlösung (Prozeßwasser) hat einen Kobaltgehalt von 0.2 bis 2.0 Gew.-%, gerechnet als Metall, und einen pH-Wert von 3 bis 4. Der pH-Wert kann beispielsweise mit Essigsäure eingestellt werden. Die Entkobaltung kann zweckmäßig bei Temperaturen von 120 bis 150 °C durchgeführt werden, um sicherzustellen, daß die im 1-Stufen-Prozeß durch Folgereaktionen gebildeten Acetale möglichst vollständig zu den gewünschten Wertprodukten Aldehyd und Alkohol zurückgespalten werden.

Die Entkobaltung wird vorzugsweise in einem mit Füllkörpern, wie z. B. Raschig-Ringen, befüllten Druckbehälter, in dem eine möglichst hohe Phasenaustauschfläche erzeugt wird, durchgeführt. Die nun von Kobaltverbindungen befreite organische Produktphase kann in einem nachgeschalteten Trennbehälter von der wäßrigen Phase getrennt werden. Die wäßrige Phase, die die aus der organischen Phase extrahierten Kobaltverbindungen z. B. in Form von Kobaltacetat oder Kobaltformiat enthält, wird vorzugsweise in den 1-Stufen-Prozeß zurückgeführt und erneut als Ausgangsstoff für die Herstellung des Kobaltkatalysators verwendet.

Geeigneterweise wird also das erfindungsgemäße Verfahren so durchgeführt, daß das Reaktionsprodukt des 1-Stufen-Prozesses zur Oxidation des Kobaltkatalysators mit Luft unter Zugabe von wäßriger, saurer Kobaltsalzlösung behandelt und nach Trennung in eine die Reaktionsprodukte enthaltende organische Phase und eine das Kobaltsalz enthaltende wäßrige Phase, die wäßrige Phase in den 1-Stufen-Prozeß zurückgeführt wird.

Anschließend kann die nach der Entfernung des Kobaltkatalysators verbleibende organische Phase hydriert und die so erhaltenen Alkohole können aus dem Hydrierprodukt z. B. durch Destillation gewonnen werden.

Unter den Reaktionsbedingungen des erfindungsgemäßen Verfahrens bilden sich neben Aldehyden durch Hydrierung auch teilweise die entsprechenden Alkohole. Die Aldehyde und Alkohole können nach der Entkobaltung aus dem organischen Reaktoraustrag abgetrennt und einzeln weiterverarbeitet werden. Vorzugsweise wird jedoch der gesamte organische Reaktoraustrag nach bekannten Verfahren, zum Beispiel durch Hydrierung und anschließende Destillation, zum entsprechenden Alkohol aufgearbeitet.

Die nach dem erfindungsgemäßen Verfähren hergestellten Alkohle eignen sich besonders als Weichmacher- und Waschmittelalkohole. Die Aldehyde können ferner zur Herstellung von Carbonsäuren verwendet werden.
Weichmacheralkohole werden durch Veresterung, z. B. mit Phthalsäureanhydrid (PSA), zu gängigen Weichmachern für Polyvinylchlorid (PVC) umgesetzt.

Mit Hilfe des erfindungsgemäßen Verfahrens lassen sich Olefine mit 6 bis 17 Kohlenstoffatomen hydroformylieren und die so erhaltenen Aldehyde hydrieren.

Das erfindungsgemäße Verfahren ist insbesondere für die Hydroformylierung von isomeren Olefingemischen geeignet, die durch Oligomerisierung von Propen und Butenen hergestellt werden. Zu den typischen Oligomerisaten, die als Rohstoffbasis für die Hydroformylierung einsetzbar sind, zählen beispielsweise Di-, Tri- und Tetra-Propen sowie Di-, Tri- und Tetra-Buten.

Vorzugsweise werden mit Hilfe des erfindungsgemäßen Verfahrens Alkohole mit 9 bis 13 C-Atomen aus den entsprechenden Olefinen hergestellt, insbesondere Isononanole aus Dibutenen.

Die Oligomerisate von n-Butenen sind über die bekannten Oligomerisierungsverfahren, z. B. über den Octol®-Prozeß von Hüls und das Dimersol®-Verfahren von IFP, großtechnisch zugänglich (J. Schulze, M. Homann : "C₄-Hydrocarbons and Derivates", S. 69 ff., Springer Verlag, Berlin/Heidelberg, 1989).

Die im erfindungsgemäßen Verfahren erhaltenen Aldehyde können nach den bekannten Hydrierverfahren in der Gas- oder Flüssigphase zu den gewünschten Alkoholen hydriert werden. (SRI International, Report No. 21 C, April 1986, S. 53 ff.) Als Katalysator für die Hydrierung von Aldehyden sind insbesondere Kupferchromit-, Nickel- und Kupfer-Zink-Katalysatoren geeignet. Zum Teil erfolgt die Hydrierung der Aldehyde zu den Alkoholen schon im 1-Stufen-Prozeß.

Die vorliegende Erfindung wird durch die nachfolgenden Beispiele näher erläutert.

### Vergleichsbeispiel: Bekanntes Verfahren zur Herstellung von Isononanol aus Dibuten

### Vorcarbonylierung :

In einem 2 l Rührautoklaven aus Edelstahl werden 1000 ml Kobaltacetathaltiges Wasser (ca. 1.0 Gew.-% Kobalt, gerechnet als Metall) vorgelegt. Unter Rühren (1000 Upm) wird in den Autoklaven Synthesegas mit einem CO/H₂-Volumenverhältnis von 1:1 bei 170 °C und 280 bar eingeleitet. Durch zeitlich versetzte Probenahme kann die Bildung der Kobaltcarbonylkomplexe während der Vorcarbonylierung analytisch verfolgt werden. Nach einer Vorcarbonylierungszeit von 6 Stunden werden ca. 65 % des eingesetzten Kobaltsalzes zu dem aktiven Kobaltkatalysator, den Kobalthydridocarbonylkomplexen, umgesetzt.
Eine deutliche Verkürzung der Vorcarbonylierungszeit kann durch Zugabe von mit Wasser nicht oder schwer mischbaren Alkoholen, wie z. B. 2-Ethylhexanol oder Isononanol, erzielt werden. Wird die Vorcarbonylierung mit einem Kobaltsalz-Wasser/Isononanol-Gemisch (250 ml Isononanol und 750 ml wässrige Kobaltacetatlösung mit 1 Gew.-% Kobalt, gerechnet als Metall) unter oben genannten Bedingungen durchgeführt, so wird ein 65%iger Umsatz des eingesetzten Kobaltsalzes zu Kobaltcarbonylkomplexen bereits nach 5 Minuten erreicht.

### Katalysatorextraktion:

Nach Beendigung der Vorcarbonylierung wird der Autoklav für die Durchführung der Katalysatorextraktion auf einen Synthesegas-Druck von 100 bar entspannt und auf eine Temperatur von ca. 60 °C abgekühlt. Unter diesen Bedingungen wird der Kobaltcarbonylkomplex nach Zugabe von 500 ml Di-n-Buten unter intensiven Rühren (1000 Upm) in das als organische Phase füngierende Di-n-Buten extrahiert. Nach einer Extraktionszeit von ca. 10 Minuten wird das Extraktionsgemisch weitere 10 Minuten zwecks Phasentrennung bei abgestelltem Rührer stehengelassen. Die olefinische Phase enthält 0.22 Gew.-% Kobalt als Kobaltcarbonylkomplex (HCo(CO)₄). Die wäßrige Phase enthält neben 0.35 Gew.-% Kobalt als Kobalt (II)-Salz zusätzlich ca. 0.57 Gew.-% Kobalt als nicht extrahierten Kobaltcarbonylkomplex. Dies bedeutet, daß nur ca. 12 % des extrahierbaren Kobaltcarbonylkomplexes in die olefinische Phase extrahiert wurden.

### Hydroformylierung :

Nach der Katalysatorextraktion wird die wäßrige Phase aus dem Autoklaven abgelassen und weitere 500 ml Di-n-Buten zugeführt. Anschließend wird die mit Kobalthydridocarbonylkomplex beladene olefinische Phase mit Synthesegas mit einem CO/H₂-Volumenverhältnis von 1:1 bei einer Temperatur von 175 °C und einem Druck von 260 bar hydroformyliert. Nach einer Reaktionszeit von 4 Stunden wird praktisch kein Synthesegas mehr aufgenommen und die Hydroformylierung ist abgeschlossen.

### Entkobaltung :

Für die Entkobaltung wird der Autoklav entspannt und das Produktgemisch auf ca. 100 °C abgekühlt. Durch Behandlung des Reaktionsgemisches mit Luft unter 100 °C in Gegenwart von verdünnter Essigsäure werden die Kobaltcarbonylkomplexe oxidiert und die resultierenden Kobaltsalze in die wäßrige Phase zurückextrahiert.

Die organische Phase weist nach einer gaschromatographischen Analyse folgende Zusammensetzung in Gew.-% auf: 12.5 % C₈-Kohlenwasserstoffe, 44.5 % Isononanale, 29.5 % Isononanole, 3.5 % Ester (Isononylfomiate), 5 % Acetale und 5 % höhersiedenden Rückstand.

### Hydrierung:

Die Isononanale und Isononylformiate werden in Gegenwart von einem Kupferchromit-Katalysator bei 20 bis 30 bar und 150 bis 250° C in der Gasphase zu den Isononanolen hydriert.

### Beispiel 1: Herstellung von Isononanolen aus Dibuten

Das erfindungsgemäße Verfahren wird in einer kontinuierlichen Versuchsanlage durchgeführt, die im wesentlichen aus einem Hochdruckrohrreaktor (90 mm Durchmesser, 3600 mm Länge) und einem nachgeschalteten, mit Raschig-Ringen befüllten Entkobaltungsbehälter (20 1 Fassungsvermögen) sowie einem Phasentrennbehälter besteht. Durch fünf, senkrecht zu der Strömungsrichtung eingebaute Lochbleche wird der Reaktorraum des Hochdruckreaktors kaskadiert. Für die Dosierung der Ausgangsstoffe wird eine 3-Stoff-Mischdüse verwendet. Der Reaktorinhalt kann je nach Bedarf über die installierten Heiz- und Kühlvorrichtungen aufgeheizt oder gekühlt werden.

Da die Vorcarbonylierung in Gegenwart eines Alkohols und/oder Aldehyds beschleunigt wird, kann zu Beginn des erfindungsgemäßen 1-Stufen-Prozesses Isononanol oder ein Isononanal/Isononanol-Gemisch als Anfahrhilfe im Reaktor vorgelegt werden. Nachdem der Reaktor auf die Betriebstemperatur von 160 bis 180 °C gebracht worden ist, werden dem Reaktor über die Mischdüse Di-n-Buten aus dem Hülser Octol-Prozeß, wäßrige Kobaltacetat-Lösung mit 1 Gew.-% Kobalt, gerechnet als Metall, und Synthesegas mit einem CO/H₂-Volumenverhältnis von 1:1 kontinuierlich zugeführt.

Folgende Durchsätze werden eingestellt: 5.0 kg/h Di-n-Buten und 0.45 kg/h Kobaltacetat-Lösung Der Reaktor wird mit Synthesegas auf einen konstanten Reaktionsdruck von 280 bar bei einem Synthesegas-Durchsatz von 2.5 bis 3.5 Nm³/h druckgeregelt. Der gewählte Di-n-Buten-Durchsatz entspricht einer auf das Reaktorvolumen bezogenen Raumzeitbelastung (LHSV) von rd. 0.3 h⁻¹ (0.3 m³ Di-n-Buten pro m³ Reaktorvolumen und Stunde)
Die organische Phase wird kontinuierlich am Kopf des Reaktors abgezogen und in die Entkobaltungsstufe auf 10 bis 15 bar entspannt. Die dem Reaktor als Kobaltacetat-Lösung zugeführte wäßrige Phase wird standgeregelt als Kobaltkomplexhaltiges Prozeßwasser am Boden des Reaktors abgezogen und ebenfalls in die Entkobaltungsstufe entspannt.
In der Entkobaltungsstufe werden beide flüssigen Phasen zusammen mit dem bei der Entspannung anfallenden Gas (nicht umgesetztes Synthesegas) bei 140 °C mit Luft oder Sauerstoff in Gegenwart von wäßriger, saurer Kobaltsalzlösung (Prozeßwasser) durch Oxidation der Kobaltcarbonylkomplexe vom Kobaltkatalysator befreit und anschließend in einem nachgeschalteten Trennbehälter getrennt. Die organische Kobaltfreie Phase wird weiter aufgearbeitet, die wäßrige Kobaltsalzlösung wird hingegen über die Mischdüse in den 1-Stufen-Prozeß zurückgeführt. Das nicht umgesetzte Synthesegas wird weiter verwendet oder verworfen.

Unter den gewählten Reaktionsbedingungen werden Di-n-Buten-Umsätze von mehr als 90 % erzielt.
Der Rohproduktaustrag nach der Entkobaltung weist nach einer gaschromatographischen Analyse folgende Zusammensetzung in Gew.-% auf: 7.0 % C₈-Kohlenwasserstoffe, 29.7 % Isononanale, 53.1 % Isononanole, 4.2 % Ester (Isononylformiate) und 6.0 % hochsiedenden Rückstand.
Der hochsiedende Rückstand kann von den Wertprodukten sehr einfach durch Destillation abgetrennt werden.
Nach der Entkobaltung wird das Rohprodukt in nachgeschalteten, bekannten Aufarbeitungsschritten durch Hydrierung und anschließende Destillation zum Isononanol (Isomerengemisch) überführt.
Die Hydrierung des Rohproduktes wird in der Gasphase bei 20 bis 25 bar und 170 bis 250° C in Gegenwart eines Kupferchromit-Katalysators durchgeführt

### Beispiel 2: Herstellung von Isotridecanol aus Tri-n-Buten

Das erfindungsgemäße Verfahren wird in der in Beispiel 1 beschriebenen Versuchsanlage auf gleiche Weise durchgeführt.

Da die Vorcarbonylierung in Gegenwart eines Alkohols und/oder Aldehyds beschleunigt wird, kann zu Beginn des erfindungsgemäßen 1-Stufen-Prozesses Iso-Tridecanol und/oder ein Iso-Tridecanol/Iso-Tridecanal-Gemisch als Anfahrhilfe im Reaktor vorgelegt werden.
Nachdem der Reaktor auf die Betriebstemperatur von 160 - 180 °C gebracht worden ist, werden dem Reaktor über die Mischdüse die Ausgangsstoffe Tri-n-Buten aus dem Hülser Octol-Prozeß, wässrige Kobaltacetat-Lösung mit 1 Gew.-% Kobalt, gerechnet als Metall, und Synthesegas mit einem CO/H₂-Volumenverhältnis von 1:1 kontinuierlich zugeführt.

Folgende Durchsätze werden eingestellt: 1.65 kg/h Tri-n-Buten und 0.15 kg/h Kobaltacetat-Lösung. Der Reaktor wird mit Synthesegas auf einen konstanten Reaktionsdruck von 280 bar bei einem Synthesegas-Durchsatz von 0.8 bis 1.2 Nm³/h druckgeregelt. Der gewählte Tri-n-Buten-Durchsatz entspricht einer auf das Reaktorvolumen bezogenen Raumzeitbelastung (LHSV) von rd. 0.1 h⁻¹ (0,1 m³ Tri-n-Buten pro m³ Reaktorvolumen und Stunde).

Der Reaktoraustrag wird, wie in Beispiel 1 beschrieben, weiter aufgearbeitet.

Unter den gewählten Reaktionsbedingungen werden Tri-n-Buten-Umsätze von mindestens 80 % erzielt.
Der Rohproduktaustrag nach der Entkobaltung weist nach einer gaschromatographischen Analyse folgende Zusammensetzung in Gew.-% auf: 16.5 % C₈-Kohlenwasserstoffe, 73.5 % Iso-Tridecanale und Iso-Tridecanole sowie 10.0 % hochsiedenden Rückstand.

Das Rohprodukt wird nach der Entkobaltung in nachgeschalteten, bekannten Aufarbeitungsschritten durch Hydrierung in Gegenwart eines Kupferchromit-Katalysators bei 20 bis 30 bar und 150 bis 250° C in der Flüssigphase und anschließende Destillation zu Iso-Tridecanolen (Isomerengemisch) umgesetzt.

## Patentansprüche

1. Verfahren zur Herstellung von Alkoholen mit 7 bis 18 C-Atomen durch Hydroformylierung der entsprechenden Olefine mit Synthesegas in Gegenwart einer einen Kobaltkatalysator enthaltenden organischen Phase bei Temperaturen von 50 bis 220° C und Drücken von 100 bis 400 bar und anschließende Hydrierung der so erhaltenen Aldehyde, wobei der Kobaltkatalysator durch Umsetzen einer wäßrigen Kobaltsalzlösung in Anwesenheit eines nicht oder nur wenig mit Wasser mischbaren organischen Lösungsmittels mit Synthesegas gebildet und die den Kobaltkatalysator enthaltende organische Phase durch Extraktion des gebildeten Kobaltkatalysators aus der wäßrigen Phase mit einem nicht oder nur wenig mit Wasser mischbaren organischen Extraktionsmittel erhalten wird,
dadurch gekennzeichnet,
daß die Bildung des Kobaltkatalysators, die Extraktion des gebildeten Kobaltkatalysators in die organische Phase und die Hydroformylierung der entsprechenden Olefine in einem 1-Stufen-Prozeß durchgeführt werden.

2. Verfahren nach Anspruch 1,
dadurch gekennzeichnet,
daß die Bildung des Kobaltkatalysators, die Extraktion des gebildeten Kobaltkatalysators in die organische Phase und die Hydroformylierung der entsprechenden Olefine in einem einzigen Reaktor durchgeführt werden.

3. Verfahren nach den Ansprüchen 1 und 2,
dadurch gekennzeichnet,
daß Alkohole mit 9 bis 13 C-Atomen aus den entsprechenden Olefinen hergestellt werden.

4. Verfahren nach Anspruch 3,
dadurch gekennzeichnet,
daß Isononanole aus Dibutenen hergestellt werden.

5. Verfahren nach den Ansprüchen 1 bis 4,
dadurch gekennzeichnet,
daß das Verfahren kontinuierlich durchgeführt wird.

6. Verfahren nach den Ansprüchen 1 bis 5,
dadurch gekennzeichnet,
daß der Reaktorraum des 1-Stufen-Prozesses durch mindestens eine Trennvorrichtung unterteilt wird.

7. Verfahren nach den Ansprüchen 1 bis 6,
dadurch gekennzeichnet,
daß die wässrige Kobaltsalzlösung, Olefin, Synthesegas, ein nicht oder nur wenig mit Wasser mischbares organisches Lösungsmittel und ein nicht oder nur wenig mit Wasser mischbares organisches Extraktionsmittel gleichzeitig in den Reaktor des 1-Stufen-Prozesses eingebracht werden.

8. Verfahren nach Anspruch 7,
dadurch gekennzeichnet,
daß die wässrige Kobaltsalzlösung, Olefin, Synthesegas, ein nicht oder nur wenig mit Wasser mischbares organisches Lösungsmittel und ein nicht oder nur wenig mit Wasser mischbares organisches Extraktionsmittel mittels einer Mischdüse in den Reaktor des 1-Stufen-Prozesses eingebracht werden.

9. Verfahren nach den Ansprüchen 1 bis 6,
dadurch gekennzeichnet,
daß ein nicht oder nur wenig mit Wasser mischbares organisches Lösungsmittel und ein nicht oder nur wenig mit Wasser mischbares organisches Extraktionsmittel im Reaktor des 1-Stufen-Prozesses vorgelegt und die wässrige Kobaltsalzlösung, Olefin und Synthesegas gleichzeitig in den Reaktor des 1-Stufen-Prozesses eingebracht werden.

10. Verfahren nach Anspruch 9,
dadurch gekennzeichnet,
daß ein nicht oder nur wenig mit Wasser mischbares organisches Lösungsmittel und ein nicht oder nur wenig mit Wasser mischbares organisches Extraktionsmittel im Reaktor des 1-Stufen-Prozesses vorgelegt und die wässrige Kobaltsalzlösung, Olefin und Synthesegas mittels einer Mischdüse in den Reaktor des 1-Stufen-Prozesses eingebracht werden.

11. Verfahren nach den Ansprüchen 1 bis 6,
dadurch gekennzeichnet,
daß ein nicht oder nur wenig mit Wasser mischbares organisches Extraktionsmittel im Reaktor des 1-Stufen-Prozesses vorgelegt wird und die wässrige Kobaltsalzlösung, Olefin, Synthesegas und ein nicht oder nur wenig mit Wasser mischbares organisches Lösungsmittel gleichzeitig in den Reaktor des 1-Stufen-Prozesses eingebracht werden.

12. Verfahren nach Anspruch 11,
dadurch gekennzeichnet,
daß ein nicht oder nur wenig mit Wasser mischbares organisches Extraktionsmittel im Reaktor des 1-Stufen-Prozesses vorgelegt wird und die wässrige Kobaltsalzlösung, Olefin, Synthesegas und ein nicht oder nur wenig mit Wasser mischbares organisches Lösungsmittel mittels einer Mischdüse in den Reaktor des 1-Stufen-Prozesses eingebracht werden.

13. Verfahren nach den Ansprüchen 1 bis 12,
dadurch gekennzeichnet,
daß das nicht oder nur wenig mit Wasser mischbare organische Lösungsmittel ein Olefin und/oder ein Aldehyd und/oder ein Alkohol ist.

14. Verfahren nach Anspruch 13,
dadurch gekennzeichnet,
daß das nicht oder nur wenig mit Wasser mischbare organische Lösungsmittel das Reaktionsprodukt aus dem 1-Stufen-Prozeß ist.

15. Verfahren nach den Ansprüchen 1 bis 14,
dadurch gekennzeichnet,
daß das nicht oder nur wenig mit Wasser mischbare organische Extraktionsmittel ein Olefin und/oder ein Aldehyd und/oder ein Alkohol ist.

16. Verfahren nach Anspruch 15,
dadurch gekennzeichnet,
daß das nicht oder nur wenig mit Wasser mischbare organische Extraktionsmittel das Reaktionsprodukt aus dem 1-Stufen-Prozeß ist.

17. Verfahren nach den Ansprüchen 1 bis 16,
dadurch gekennzeichnet,
daß das nicht oder nur wenig mit Wasser mischbare Lösungsmittel und das nicht oder nur wenig mit Wasser mischbare Extraktionsmittel identisch sind.

18. Verfahren nach den Ansprüchen 1 bis 17,
dadurch gekennzeichnet,
daß die wäßrige Kobaltsalzlösung, Olefin und Synthesegas gleichzeitig in den Reaktor des 1-Stufen-Prozesses eingebracht werden.

19. Verfahren nach Anspruch 18,
dadurch gekennzeichnet,
daß die wäßrige Kobaltsalzlösung, Olefin und Synthesegas mittels einer Mischdüse in den Reaktor des 1-Stufen-Prozesses eingebracht werden.

20. Verfahren nach den Ansprüchen 1 bis 19,
dadurch gekennzeichnet,
daß das Reaktionsprodukt des 1-Stufen-Prozesses zur Oxidation des Kobaltkatalysators mit Luft unter Zugabe von wäßriger, saurer Kobaltsalzlösung behandelt wird und nach Trennung in eine die Reaktionsprodukte enthaltende organische Phase und eine das Kobaltsalz enthaltende wäßrige Phase die wäßrige Phase in den 1-Stufen-Prozeß zurückgeführt wird.

21. Verfahren nach den Ansprüchen 1 bis 20,
dadurch gekennzeichnet,
daß die nach der Entfernung des Kobaltkatalysators verbleibende organische Phase hydriert wird und die so erhaltenen Alkohole aus dem Hydrierprodukt gewonnen werden.

## Claims

1. A process for the production of alcohols having 7 to 18 C atoms by hydroformylation of the corresponding olefins with synthesis gas in the presence of an organic phase containing a cobalt catalyst at temperatures from 50 to 220°C and pressures from 100 to 400 bar and subsequent hydrogenation of the aldehydes thus obtained, the cobalt catalyst being formed by reacting an aqueous cobalt salt solution with synthesis gas in the presence of an organic solvent which is only slightly miscible or not miscible at all with water, and the organic phase containing the cobalt catalyst being obtained by extraction of the cobalt catalyst formed from the aqueous phase by means of an organic extractant which is only slightly miscible or not miscible at all with water,
characterized in that the formation of the cobalt catalyst, the extraction of the cobalt catalyst formed into the organic phase and the hydroformylation of the corresponding olefins are carried out in a 1-stage process.

2. A process according to claim 1, characterized in that the formation of the cobalt catalyst, the extraction of the cobalt catalyst formed into the organic phase and the hydroformylation of the corresponding olefins are carried out in one single reactor.

3. A process according to either of claims 1 and 2, characterized in that alcohols having 9 to 13 C atoms are produced from the corresponding olefins.

4. A process according to claim 3, characterized in that isononanols are produced from dibutenes.

5. A process according to any of claims 1 to 4, characterized in that the process is carried out continuously.

6. A process according to any of claims 1 to 5, characterized in that the reactor space of the 1-stage process is subdivided by at least one partition device.

7. A process according to any of claims 1 to 6, characterized in that the aqueous cobalt salt solution, olefin, synthesis gas, an organic solvent which is only slightly miscible or not miscible at all with water, and an organic extractant which is only slightly miscible or not miscible at all with water are simultaneously fed to the reactor of the 1-stage process.

8. A process according to claim 7, characterized in that the aqueous cobalt salt solution, olefin, synthesis gas, an organic solvent which is only slightly miscible or not miscible at all with water, and an organic extractant which is only slightly miscible or not miscible at all with water are fed to the reactor of the 1-stage process by means of a mixing nozzle.

9. A process according to any of claims 1 to 6, characterized in that an organic solvent which is only slightly miscible or not miscible at all with water, and an organic extractant which is only slightly miscible or not miscible at all with water, are initially introduced into the reactor of the 1-stage process, and the aqueous cobalt salt solution, olefin and synthesis gas are simultaneously fed to the reactor of the 1-stage process.

10. A process according to claim 9, characterized in that an organic solvent which is only slightly miscible or not miscible at all with water, and an organic extractant which is only slightly miscible or not miscible at all with water, are initially introduced into the reactor of the 1-stage process, and the aqueous cobalt salt solution, olefin and synthesis gas are fed to the reactor of the 1-stage process by means of a mixing nozzle.

11. A process according to any of claims 1 to 6, characterized in that an organic extractant which is only slightly miscible or not miscible at all with water is initially introduced into the reactor of the 1-stage process, and the aqueous cobalt salt solution, olefin, synthesis gas and an organic solvent which is only slightly miscible or not miscible at all with water are fed simultaneously to the reactor of the 1-stage process.

12. A process according to claim 11, characterized in that an organic extractant which is only slightly miscible or not miscible at all with water is initially introduced into the reactor of the 1-stage process and the aqueous cobalt salt solution, olefin, synthesis gas and an organic solvent which is only slightly miscible or not miscible at all with water are fed to the reactor of the 1-stage process by means of a mixing nozzle.

13. A process according to any of claims 1 to 12, characterized in that the organic solvent which is only slightly miscible or not miscible at all with water is an olefin and/or an aldehyde and/or an alcohol.

14. A process according to claim 13, characterized in that the organic solvent which is only slightly miscible with water or not miscible at all with water is the reaction product from the 1-stage process.

15. A process according to any of claims 1 to 14, characterized in that the organic extractant which is only slightly miscible or not miscible at all with water is an olefin and/or an aldehyde and/or an alcohol.

16. A process according to claim 15, characterized in that the organic extractant which is only slightly miscible or not miscible at all with water is the reaction product from the 1-stage process.

17. A process according to any of claims 1 to 16, characterized in that the solvent which is only slightly miscible or not miscible at all with water and the extractant which is only slightly miscible or not miscible at all with water are identical.

18. A process according to any of claims 1 to 17, characterized in that the aqueous cobalt salt solution, olefin and synthesis gas are simultaneously fed to the reactor of the 1-stage process.

19. A process according to claim 18, characterized in that the aqueous cobalt salt solution, olefin and synthesis gas are fed to the reactor of the 1-stage process by means of a mixing nozzle.

20. A process according to any of claims 1 to 19, characterized in that the reaction product of the 1-stage process is treated with air, with addition of aqueous acidic salt solution, for oxidizing the cobalt catalyst and, after separation into an organic phase containing the reaction products and an aqueous phase containing the cobalt salt, the aqueous phase is recycled to the 1-stage process.

21. A process according to any of claims 1 to 20, characterized in that the organic phase remaining after the removal of the cobalt catalyst is hydrogenated and the alcohols thus obtained are recovered from the hydrogenation product.

## Revendications

1. Procédé de préparation d'alcools ayant de 7 à 18 atomes de carbone par hydroformylation des oléfines correspondantes à l'aide de gaz de synthèse en présence d'une phase organique contenant un catalyseur au cobalt à des températures allant de 50 à 220°C et à des pressions allant de 100 à 400 bars, et hydrogénation consécutive de l'aldéhyde ainsi obtenu, dans lequel le catalyseur au cobalt est formé par réaction d'une solution aqueuse de cobalt en présence d'un solvant organique non miscible à l'eau ou seulement faiblement miscible à l'eau, avec du gaz de synthèse et la phase organique contenant le catalyseur au cobalt, est obtenue par extraction du catalyseur au cobalt formé, de la phase aqueuse avec un agent d'extraction organique non miscible ou seulement faiblement miscible à l'eau,
caractérisé en ce que
la formation du catalyseur au cobalt, l'extraction du catalyseur au cobalt formé dans la phase organique et l'hydroformylation de l'oléfine correspondante sont effectués dans un processus à une étape.

2. Procédé selon la revendication 1,
caractérisé en ce que
la formation du catalyseur au cobalt, l'extraction du catalyseur au cobalt formé dans la phase organique et l'hydroformylation de l'oléfine correspondante sont effectués dans un réacteur unique.

3. Procédé selon les revendications 1 et 2,
caractérisé en ce que
des alcools ayant de 9 à 13 atomes de carbone sont préparés à partir des oléfines correspondantes.

4. Procédé selon la revendication 3,
caractérisé en ce que
des isononanols sont préparés à partir de dibutènes.

5. Procédé selon l'une quelconque des revendications 1 à 4,
caractérisé en ce que
le procédé est exécuté en continu.

6. Procédé selon l'une quelconque des revendications 1 à 5,
caractérisé en ce que
l'espace du réacteur du processus à une étape est divisé par au moins un dispositif de séparation,

7. Procédé selon l'une quelconque des revendications 1 à 6,
caractérisé en ce que
la solution aqueuse de sel de cobalt, l'oléfine, le gaz de synthèse, un solvant organique non miscible à l'eau ou seulement faiblement miscible à l'eau, et un agent d'extraction organique non miscible à l'eau ou faiblement miscible à l'eau sont introduits simultanément dans le réacteur du processus à une étape.

8. Procédé selon la revendication 7,
caractérisé en ce que
la solution aqueuse de sel de cobalt, l'oléfine, le gaz de synthèse, un agent d'extraction organique non miscible à l'eau ou seulement faiblement miscible à l'eau et un agent d'extraction organique non miscible à l'eau ou faiblement miscible à l'eau sont introduits à l'aide d'une buse de mélange dans le réacteur du processus à une étape.

9. Procédé selon l'une quelconque des revendications 1 à 6,
caractérisé en ce qu'
un solvant organique non miscible à l'eau ou seulement faiblement miscible à l'eau et un agent d'extraction organique non miscible à l'eau ou seulement faiblement miscible à l'eau sont introduits dans le réacteur du processus à une étape et la solution aqueuse de sel de cobalt, l'oléfine et le gaz de synthèse sont placés simultanément dans le réacteur de processus à une étape.

10. Procédé selon la revendication 9,
caractérisé en ce qu'
un solvant organique non miscible à l'eau ou seulement faiblement miscible à l'eau, un agent d'extraction organique non miscible à l'eau ou seulement faiblement miscible à l'eau, introduits dans le réacteur du processus à une étape et la solution aqueuse de sel de cobalt, l'oléfine et le gaz de synthèse sont placés à l'aide d'une buse de mélange dans le réacteur de processus à une étape.

11. Procédé selon l'une quelconque des revendications 1 à 6,
caractérisé en ce qu'
un agent d'extraction organique non miscible à l'eau ou seulement faiblement miscible à l'eau, est introduit dans le réacteur de processus à une étape et la solution aqueuse de sel de cobalt, l'oléfine et le gaz de synthèse et un solvant organique non miscible à l'eau ou seulement faiblement miscible à l'eau sont placés simultanément dans le réacteur de processus à une étape.

12. Procédé selon la revendication 11,
caractérisé en ce qu'
un agent d'extraction organique non miscible à l'eau ou seulement faiblement miscible à l'eau est introduit dans le réacteur du processus à une étape, et la solution aqueuse de sel de cobalt, l'oléfine, le gaz de synthèse et un solvant organique non miscible à l'eau ou seulement faiblement miscible à l'eau, sont placés dans le réacteur du processus à une étape à l'aide d'une buse de mélange.

13. Procédé selon l'une quelconque des revendications 1 à 12,
caractérisé en ce que
le solvant organique non miscible à l'eau ou seulement faiblement miscible à l'eau est une oléfine et/ou un aldéhyde et/ou un alcool.

14. Procédé selon la revendication 13,
caractérisé en ce que
le solvant organique non miscible à l'eau ou seulement faiblement miscible à l'eau est le produit de réaction provenant du processus à une étape.

15. Procédé selon l'une quelconque des revendications 1 à 14,
caractérisé en ce que
l'agent d'extraction organique non miscible à l'eau ou seulement faiblement miscible à l'eau est une oléfine et/ou un aldéhyde et/ou un alcool,

16. Procédé selon la revendication 15,
caractérisé en ce que
l'agent d'extraction organique non miscible à l'eau ou seulement faiblement miscible à l'eau est le produit de réaction provenant du processus à une étape.

17. Procédé selon l'une quelconque des revendications 1 à 16,
caractérisé en ce que le solvant non miscible à l'eau ou seulement faiblement miscible à l'eau et l'agent d'extraction non miscible à l'eau ou seulement faiblement miscible à l'eau sont identiques.

18. Procédé selon l'une quelconque des revendications 1 à 17,
caractérisé en ce que
la solution aqueuse de sel de cobalt, l'oléfine, le gaz de synthèse sont introduits simultanément dans le réacteur du processus à une étape.

19. Procédé selon la revendication 18,
caractérisé en ce que
la solution aqueuse de sel de cobalt, l'oléfine et le gaz de synthèse sont introduits dans le réacteur du processus à une étape à l'aide d'une buse de mélange.

20. Procédé selon l'une quelconque des revendications 1 à 19,
caractérisé en ce que
le produit de réaction du processus à une étape est traité en vue de l'oxydation du catalyseur au cobalt, avec de l'air en ajoutant une solution aqueuse, acide, de sel de cobalt, et, après séparation en une phase organique contenant les produits de réaction et en une phase aqueuse contenant le sel de cobalt, la phase aqueuse est ramenée dans le processus à une étape.

21. Procédé selon l'une quelconque des revendications 1 à 20.
caractérisé en ce que
la phase organique qui demeure après l'élimination du catalyseur au cobalt, est hydrogénée et les alcools ainsi obtenus sont récupérés du produit d'hydrogénation.
